# EUROPÄISCHE PATENTANMELDUNG

(11) **EP 2 181 700 A1**
(43) Veröffentlichungstag der Anmeldung: **05.05.2010**
(21) Anmeldenummer: 09012861.2
(22) Anmeldetag: 12.10.2009
(51) Int. Cl.: A61K 8/895, A61Q 1/02, A61Q 1/04, A61Q 1/10

(54) **Kosmetische Zubereitung mit Silikon-Acrylat-Copolymeren**

(30) Priorität: 30.10.2008 DE 102008056373
(71) Anmelder: BEIESRDORF AG, 20245 Hamburg (DE)
(72) Erfinder: Münchow, Lynn, 22547 Hamburg (DE); Lemmer, Katharina, 20259 Hamburg (DE); Heuer, Björn, Dr., 22145 Hamburg (DE); Kummer, Andreas B., Dr., 21079 Hamburg (DE)

(57) **Zusammenfassung**

Kosmetische Zubereitung enthaltend mindestens zwei strukturell unterschiedliche Silikon-AcrylatCopolymere.

## Beschreibung

Die vorliegende Erfindung betrifft eine kosmetische Zubereitung enthaltender mindestens zwei strukturell unterschiedlicher Silikon-Acrylat-Copolymere.

Der Wunsch, schön und attraktiv auszusehen, ist seit Tausenden von Jahren in den Menschen verwurzelt. Auch wenn das Schönheitsideal im Laufe der Zeit Wandlungen erfahren hat, so ist das Streben nach einem makellosen Äußeren, immer das Ziel der Menschen gewesen, da ein sympathisches Erscheinungsbild ihr Selbstwertgefühl und die Anziehungskraft auf ihre Mitmenschen erhöht.

Der Begriff der dekorativen Kosmetik leitet vom lateinischen "decoratio" - das Hervorheben des Schönen - ab. Meist werden dabei mit Hilfe von Farbstoffen einzelne Körperpartien, insbesondere im Gesicht, hervorgehoben und farbliche Uneinheitlichkeiten abgemildert.

Das Gesichts-Make-up soll der Gesichtshaut ein natürliches Aussehen verleihen, blasse Haut auffrischen und farbliche Unregelmäßigkeiten der Haut ausgleichen.

Neben Gesichtspudern und Rouge als pulverförmigen Kosmetika, werden hierzu cremeförmige Präparate, wie Tagescremes und Creme-Make-up auf Emulsionsbasis verwendet. Eine besondere Ausführungsform ist die sogenannte Foundation. Als Foundation bezeichnet man dabei flüssige oder halbfeste Make-up-Präparate, die meist hautfarben sind (d.h. die Haut tönen) und auf das Gesicht, insbesondere auf die Wangen aufgetragen werden. Sie verleihen diesen ein gleichmäßiges gesundes gebräuntes oder rötliches Aussehen.

Nachteilig am Stande der Technik ist der Umstand, dass herkömmliche Foundations auf der Gesichtshaut nur begrenzt haltbar sind. Im Verlauf der Tragezeit kommen Unregelmäßigkeiten der Haut häufig wieder zum Vorschein. Herkömmliche Foundations (insbesondere W/S Foundations) können darüber hinaus keine ausreichende Haftfestigkeit gewährleisten, die über einen ganzen Tag (24h) anhält. Ferner lassen sich herkömmliche Foundations allzu leicht durch das unbeabsichtigte Berühren der Haut mit den Fingern oder einem Taschentuch, abtragen. Das Hautbild sieht dadurch nicht mehr ebenmäßig und perfekt aus. Dieser Effekt wird durch starke Sebumproduktion oder schweißtreibende Aktivität zusätzlich verstärkt.

Ein weiterer Nachteil herkömmlicher Foundations besteht darin, dass die Haut nach einer gewissen Tragezeit ein fettig-glänzendes Aussehen bekommt. Dieser Effekt tritt insbesondere in den so genannten T-Zonen des Gesichtes und besonders bei Hauttypen mit hoher Sebumproduktion auf. Unregelmäßigkeiten, wie kleine Pickelchen oder Couperose, treten dann noch deutlicher in Erscheinung.

Nachteilig am Stande der Technik ist nicht zuletzt der Umstand, dass herkömmliche Foundations nach dem Auftragen nicht besonders feuchtigkeits- und hitzestabil sind. Insbesondere bei sportlichen Aktivitäten "verrutscht" und verschmiert das Make-up in unästhetischer Weise. Statt Ungleichmäßigkeiten im Erscheinungsbild der Haut zu glätten, werden die Unebenheiten eher noch verstärkt.

Es war daher die Aufgabe der vorliegenden Erfindung, die Nachteile des Standes der Technik zu beseitigen.

Darüber hinaus war es die Aufgabe der vorliegenden Erfindung, eine Foundation zu entwickeln, die sich einfach und kostengünstig herstellen lässt und eine angenehme Sensorik aufweist.

Überraschend gelöst werden die Aufgaben durch eine kosmetische Zubereitung enthaltend mindestens zwei strukturell unterschiedliche Silikon-Acrylat-Copolymere, sowie durch die Verwendung mindestens zweier strukturell unterschiedlicher Silikon-Acrylat-Copolymere in kosmetischen Zubereitungen zur Erhöhung der Haftfestigkeit der Zubereitung auf der Haut.

Zwar kennt der der Fachmann als Stand der Technik die EP 0963751 sowie die DE 10135520. doch konnten diese Schriften nicht den Weg zur vorliegenden Erfindung weisen.

Die Formulierungen "erfindungsgemäß", "erfindungsgemäß vorteilhaft" etc. beziehen sich im Rahmen der vorliegenden Offenbarung sowohl auf die erfindungsgemäße Zubereitung als auch auf die erfindungsgemäße Verwendung.

Es ist erfindungsgemäß bevorzugt, wenn Zubereitung zwei strukturell unterschiedliche Silikon-Acrylat-Copolymere enthält,

Es ist erfindungsgemäß vorteilhaft, wenn die erfindungsgemäße Zubereitung in Form einer Emulsion vorliegt.

Es ist erfindungemäß bevorzugt, wenn die Zubereitung in Form einer Wasser-in-Silikonöl-Emulsion vorliegt.

Es ist erfindungsgemäß vorteilhaft, wenn eines der Silikon-Acrylat-Copolymere ein Pfropf-Copolymer darstellt.

Stellt eines der Silikon-Acrylat-Copolymere eine Pfropf-Copolymer dar, so ist es erfindungsgemäß vorteilhaft, wenn das Pfropf-Copolymer ein Acrylat-Rückgrat und Silikon-Seitenketten aufweist. Erfindungsgemäß bevorzugt ist es, als Pfropf-Copolymer das Polymer i mit der INCI Acrylates/Dimethicone Copolymer einzusetzen.

Es ist erfindungsgemäß vorteilhaft, wenn eines der Silikon-Acrylat-Copolymere ein Copolymer aus (Meth)Acrylsäure und/oder Alkyl(meth)acrylaten mit einem Acrylatester eines dendrimeren Silikons darstellt.

In einem solchen Falle ist es erfindungsgemäß vorteilhaft, wenn als Copolymer aus (Meth)Acrylsäure und/oder Alkyl(meth)acrylaten mit einem Acrylatester eines dendrimeren Silikon die Verbindung mit der CAS-Nummer 262299-63-8 eingesetzt wird, welche die INCI Acrylatas/Polytrimethylsiloxymethacrylate Copolymer trägt.

Erfindungsgemäß bevorzugt sind also insbesondere Zubereitungen und Verwendungen, die Silikon/Acrylat-Copolymere mit der INCI Acrylates/Dimethicone Copolymer und Acrylates/Polytrimethylsiloxymethacrylate Copolymer enthalten.

Acrylates/Dimethicone Copolymer kann beispielsweise unter der Handelsbezeichnung KP-545 oder KP-550 bei der Firma Shin-Etsu erworben werden.

Acrylates/Polytrimethylsiloxymethacrylate Copolymer kann beispielsweise bei der Firma Dow Coming unter der Bezeichnung FA 4001 CM oder FA 4002 ID erworben werden.

Vorteilhafte Ausführungsformen der vorliegenden Erfindung sind **dadurch gekennzeichnet, dass** das Gewichtsverhältnis von Copolymer aus (Meth)Acrylsäure und/oder Alkyl(meth)acrylaten mit einem Acrylatester eines dendrimeren Silikon zu Pfropf-Copolymer 1:4 beträgt wobei leichte Abweichungen von diesem Gewichtsverhältnis ebenfalls als erfindungsgemäß anzusehen sind.

Erfindungsgemäß besonders bevorzugt sind also Zubereitungen und Verwendungen, die Silikon/Acrylat-Copolymere mit der INCI Acrylates/Dimethicone Copolymer und Acrylates/Polytrimethylsiloxymethacrylate Copolymer im Gewichtsverhältnis 1:4 enthalten, wobei leichte Abweichungen von diesem Gewichtsverhältnis ebenfalls als erfindungsgemäß anzusehen sind.

Erfindungsgemäß vorteilhafte Ausführungsformen der vorliegenden Erfindung sind **dadurch gekennzeichnet, dass** die Gesamtmenge an Silikon-Acrylat-Copolymeren in der Zubereitung von 0,5 bis 15 Gewichts-%, bezogen auf das Gesamtgewicht der Zubereitung, beträgt.

Erfindungsgemäß vorteilhaft wird dabei das Pfropf-Copolymer (insbesondere Acrylates/Dimethicone Copolymer) in einer Menge von 0,3 bis 12 Gewichts-%, bezogen auf das Gesamtgewicht der Zubereitung einzusetzen.

Die erfindungsgemäß vorteilhafte Einsatzkonzentration von Copolymer aus (Meth)Acrylsäure und/oder Alkyl(meth)acrylaten mit einem Acrylatester eines dendrimeren Silikon beträgt, bezogen auf das Gesamtgewicht der Zubereitung, von 0,1 bis 5 Gewichts-%.

Es ist erfindungsgemäß bevorzugt, wenn die erfindungsgemäße Zubereitung Cyclomethicone und/oder Isododecan enthält.

Neben diesen erfindungsgemäß bevorzugten Ölkomponenten, kann die erfindungsgemäße Zusammensetzung aber auch weitere Ölkomponenten, beispielsweise Dimethicon, Dimethiconol, Dicaprylylcarbonat, Dicaprylylether, Myristyl Lactat, Hydrogenated Polyisobuten, Polydecen, Trimethylsiloxysilikat, Squalan, Propylencarbonat und/oder Polyethylen/Dimethiconol Copolymer, enthalten.

Erfindungsgemäße Ausführungsformen der vorliegenden Erfindung sind auch **dadurch gekennzeichnet, dass** sie ein oder mehrere Farbpigmente gewählt aus der Gruppe der Verbindungen mit dem Color Index CI 77491, CI 77492, CI 77499, CI, 77007, CI 77891 enthält.

Es ist vorteilhaft im Sinne der vorliegenden Erfindung, wenn die Farbstoffe und/oder Farbpigmente gewählt werden aus der Gruppe der Verbindungen mit dem Color-Index CI 77007, CI 77491, CI 77492, CI 77499, CI77891. Diese vorteilhaften Farbpigmente werden erfindungsgemäß vorteilhaft in einer Gesamtkonzentration von 1- 20 Gew.-% und bevorzugt in einer Gesamtkonzentration von 5 -15 Gew-%, bezogen auf das Gesamtgewicht der Zubereitung eingesetzt.

Es kann erfindungsgemäß von Vorteil sein, wenn die erfindungsgemäße Zubereitung WeißPigmente gewählt aus der Gruppe der Verbindungen Kaolin, Titandioxid und Zinkoxid enthält.

In einem solchen Falle ist es erfindungsgemäß von Vorteil, wenn die Gesamtmenge an Weiß-Pigmenten von 2 bis 12 Gew.-%, bezogen auf das Gesamtgewicht der Zubereitung, beträgt.

Es ist erfindungsgemäß vorteilhaft, wenn die Zubereitung Farbstoffe und Farbpigmente in einer Gesamtkonzentration von 5 bis 15 Gew.-%, bezogen auf das Gesamtgewicht der Zubereitung, enthält.

Es ist erfindungsgemäß vorteilhaft, wenn die Zubereitung W/S-Emulgatoren gewählt aus der Gruppe der Verbindungen Cetyl PEG/PPG-10/1 Dimethicone oder PEG/PPG-19/19 Dimethicone enthält.

Erfindungsgemäß vorteilhafte Ausführungsformen sind auch **dadurch gekennzeichnet, dass** die Zubereitung einen oder mehrere Füllstoffe gewählt aus der Gruppe der Verbindungen Disteardimoniumhectorid, Nylon 6/12, Silica, Aluminiumstärkeoctenylsuccinate. Dimethicon-Crosspolymer, Dimethicon/Vinyldimethicon-Crosspolymer, Methylmethacrylat-Crosspolymer, Lauroyl Lysine, Polymethylsilsequioxan enthält.

Es ist außerdem vorteilhaft im Sinne der vorliegenden Erfindung, wenn die Zubereitung Natriumchlorid enthält.

Erfindungsgemäß vorteilhaft ist es, wenn die Zubereitung ein oder mehrere Antioxidantien gewählt aus der Gruppe der Verbindungen Tocopherolacetat, Tocopherol, 2,6-Di-*tert*-butyl-4-methylphenol enthält.

Vorteilhafte Ausführungsformen der vorliegenden Erfindung sind ferner **dadurch gekennzeichnet, dass** die Zubereitung in der wässrigen Phase ein oder mehrere Verbindungen gewählt aus der Gruppe der Verbindungen Glycerin, Ethanol, Butylenglycol, Wasser, Lecithin enthält.

Nicht zuletzt enthält die erfindungsgemäße Zubereitung vorteilhaft ein oder mehrere Parfümstoffe, UV-Filter (ein oder mehrere UV-A- und/oder UV-B-Filter) und/oder Konservierungsmittel, beispielsweise Parabene, Phenoxyethanol etc.

Ansonsten unterscheiden sich die erfindungemäßen Zusammensetzungen nicht wesentlich von den herkömmlichen, bekannten Zubereitungen (insbesondere Foundations).

Es ist erfindungsgemäß bevorzugt, wenn die Zubereitung eine Foundation darstellt, Aber auch der Einsatz in anderen kosmetischen Zubereitungen ist als erfindungsgemäß anzusehen.

### Vergleichsversuche

Die folgenden Experimente können die erfindungsgemäßen Eigenschaften der vorliegenden Erfindung verdeutlichen (ohne dass die Erfindung darauf beschränkt wäre):

| | **A Vergleich** | **B Vergleich** | **C Vergleich** | **D Erfindung** |
|---|---|---|---|---|
| Aqua | add 100 | add 100 | add 100 | add 100 |
| Glycerin | 5 | 5 | 5 | 5 |
| CI 77891 + CI 77492 + CI 77491 + CI 77499 | 7 | 7 | 7 | 7 |
| Sodium Chloride | 2,5 | 2,5 | 2,5 | 2,5 |
| Diazolidinyl Urea | 0,3 | 0,3 | 0,3 | 0,3 |
| | | | | |
| Cetyl PEG/PPG-10/1 Dimethicone | 4 | 4 | 4 | 4 |
| Dicaprylyl Carbonate | 5 | 5 | 5 | 5 |
| Cyclomethicone | 17 | 7 | 7 | 7 |
| KP-545 Cyclomethicone + Acrylates/ Dimethicone Copolymer | | 15 | | 12 |
| FA 4001 Cyclomethicone + Acrylates/Polytrimethylsiloxymethacrylate Copolymer | | | 15 | 3 |
| Trimethylslioxysilicate | 5 | | | |
| Parfum | 0,4 | 0,4 | 0,4 | 0,4 |
| Talk | 1 | 1 | 1 | 1 |
| Silica Dimethyl Silylate | 0,5 | 0,5 | 0,5 | 0,5 |
| Lauroyl Lysine | 3 | 3 | 3 | 3 |
| Nylon 6/12 | 2 | 2 | 2 | 2 |
| Ronaspheren LDP Silica + CI 77891 + CI 77491 | 3 | 3 | 3 | 3 |

| | | | | |
|---|---|---|---|---|
| Herstellung: Wasserphase unter Rühren in die Ölphase geben. Nach der Emulgierung Füllstoffe zufügen. | | | | |

Anhand der angeführten Vergleichsformulierungen lässt sich der Vorteil der erfindungsgemäßen Kombination von mindestens zwei strukturell unterschiedlichen Silikon-Acrylat-Copolymeren erkennen. Beispiel A entspricht dem Stand der Technik und zeigt die bekannte Hauthaftung von W/S Foundations gänzlich ohne filmbildendes Polymer. Das erfindungsgemäße Beispiel D jedoch zeigt im Anwendungstest mit 8 Probanden über 8 Stunden signifikant bessere Eigenschaften in punkto Hauthaftung, gleichmäßiges Hautbild über den Tag und Abdecken von Unreinheiten, Diese können nicht durch den Einsatz eines der beiden Silikon-Acrylat-Copolymere allein erzielt werden. Beispiel B hinterlässt einen unangenehm klebrigen und glänzenden Film auf der Haut, der über den Tag "verrutscht". Beispiel C bildet einen unangenehm spröden und "spannenden" Film auf der Haut, der über den Tag "bröckelt".

### Beispiele

Die nachfolgenden Beispiele sollen die vorliegende Erfindung verdeutlichen, ohne sie einzuschränken. Alle Mengenangaben, Anteile und Prozentanteile sind, soweit nicht anders angegeben, auf das Gewicht und die Gesamtmenge bzw. auf das Gesamtgewicht der Zubereitungen bezogen.

W/S Foundations:

| | 1 | 2 | 3 | 4 |
|---|---|---|---|---|
| | Gew.-% | Gew.-% | Gew.-% | Gew.-% |
| Wasser | Ad 100 | Ad 100 | Ad 100 | Ad 100 |
| Glycerin | 7 | 5 | 5 | 10 |
| Cyclomethicon + PEG/PPG-19/19 Dimethicon | | 4 | 2 | |
| Cetyl PEG/PPG-10/1 Dimethicon | 4 | | | 5 |
| Propylene Carbonat | 0,05 | 0,06 | 0,06 | 0,03 |
| Disteardimonium Hectorit | 0,25 | 0,4 | 0,2 | 0,1 |
| Natrium Chlorid | 2 | | 1 | 2 |
| Squalan | 0,5 | | 0,5 | |
| Dicaprylyl Carbonat | | 5 | | 5 |
| Cyclomethicon | 10 | 12 | 20 | 15 |
| Dicaprylyl Ether | 2 | | | |
| Caprytic/Capnc Triglycerid | 2 | | | |
| Dimethicon | 2 | 3 | | 4 |
| Acrylates/Polytrimethylsiloxymethacrylate Copolymer | 3 | 1,5 | 0,5 | 2 |
| Acrylates/Dimethicone Copolymer | 9 | 5 | 1,5 | 5,5 |
| Myristyl Laktat | | | 1 | |
| Lecithin | 1 | | 0,5 | |
| Trimethylsiloxysilikat | | | 3,5 | 2 |
| Lauroyl Lysine | 5 | | 2,5 | 3 |
| Talkum | 2 | 3 | 0,5 | |
| Nylon 6/12 | | 3 | 2 | 4 |
| Silica | | 3 | 4 | 3 |
| Dimethicon Crosspolymer | 2 | 1 | | |
| Methyl Methacrylat Crosspolymer | | | 1 | 2 |
| Polymethylsilsesquioxan | 2 | 1 | | |
| VPNA Copolymer | 0,1 | | | 0,2 |
| VP/Hexadecen Copolymer | 0,1 | | 0,5 | |
| Titandioxid (CI 77891) | 6 | 3 | 3 | 8 |
| Farbpigmente (CI 77492 + CI 77491 + CI 77499) | 5 | 5 | 2 | 7 |
| Effektpigmente (z.B. beschichtetes Mica) | | 3 | 1 | |
| Titandioxid | 1 | 2 | | |
| Dimethicodiethylbenzalmalonat (PolysiliCOne-15) | 0,5 | | 4 | 2,5 |
| 2-Phenylbenzimidazol-5-sulfonsäuresalze | 1 | | 2 | 3 |
| Sodium Ascorbyl Phosphate | | | 0,1 | 0,2 |
| Tocopheryl Acetate | 0,5 | 0,5 | 1 | 1,5 |
| Ubiquinon, Q10 | | 0,05 | | |
| Phenoxyethanol + Parabene | 1 | 0,7 | | |
| Diazolidinyl Urea | | | 0,3 | 0,3 |
| Parfum | 0,5 | 0,5 | 0,5 | 0,5 |

**Wasserfeste Maskara:**

| | 5 | 6 | 7 |
|---|---|---|---|
| | Gew.-% | Gew.-% | Gew.-% |
| Quatemium-18-Hectorite | 10,00 | 12,00 | 10,00 |
| Talkum | 5,00 | 4,00 | 5,00 |
| Ethanol | 2,50 | 2,75 | 2,50 |
| Lycra ® | 0,05 | 0,40 | 1,00 |
| Carnaubawachs | 2,00 | - | 3,00 |
| PVP/Eicosencopolymer | 20,00 | 18,00 | 17,00 |
| Perlglanzpigmente | 5,00 | 3,00 | 3,00 |
| Farbpigmente(Eisenoxide) | 10,00 | 10,00 | 10,00 |
| Nylon-6 | - | 2,00 | 1,00 |
| Cyclomethicon | 5,00 | - | 3,00 |
| Acrylates/Polytrimethylsilo xymethacrylate Copolymer | 1 | 3,5 | 2 |
| Acrylates/Dimethicone Copolymer | 3 | 11,5 | 5 |
| Konservierungsmittel | q.s. | q.s. | q.s. |
| Caiciumpanthotenat | 0,80 | 0,10 | 0,20 |
| Gamma-Oryzanol | 0,20 | 5,00 | 1,00 |
| Isoparaffin C₁₀₋₁₂ | ad 100,00 | ad 100,00 | ad 100,00 |

**Lippenfarbe:**

| | 8 | 9 |
|---|---|---|
| | Gew.% | Gew.% |
| Acrylates/Polytrimethylsiloxymethacrylate Copolymer | 3 | 1,5 |
| Acrylates/Olmethicone Copolymer | 9 | 4,5 |
| Isoparaffin C₁₀₋₁₂ | ad 100 | ad 100 |
| Synthetic Wax | | 10 |
| Sunflower Wax | | 4 |
| Pigments | 10 | 10 |
| Sucrose Acetate Ester | 0,5 | 2 |
| Propylparaben | 0,1 | 0,1 |
| BHT | 0,03 | 0,03 |
| Polyisobutene | 5 | |
| Hydrogenated Polydecene | | 10 |
| Glimmer | 5 | 5 |
| Jojoba oil | 2 | |

## Patentansprüche

1. Kosmetische Zubereitung enthaltend mindestens zwei strukturell unterschiedliche Silikon-Acrylat-Copolymere.

2. Verwendung mindestens zweier strukturell unterschiedlicher Silikon-Acrylat-Copolymere in kosmetischen Zubereitungen zur Erhöhung der Haftfestigkeit der Zubereitung auf der Haut.

3. Kosmetische Zubereitung nach Anspruch 1 oder Verwendung nach Anspruch 2, **dadurch gekennzeichnet, dass** die Zubereitung in Form einer Emulsion vorliegt.

4. Kosmetische Zubereitung oder Verwendung nach einem der vorhergehenden Ansprüche, **dadurch gekennzeichnet, dass** die Zubereitung in Form einer Wasser-in-Silikonöl-Emulsion vorliegt.

5. Kosmetische Zubereitung oder Verwendung nach einem der vorhergehenden Ansprüche, **dadurch gekennzeichnet, dass** eines der Silikon-Acrylat-Copolymere ein Pfropf-Copolymer darstellt.

6. Kosmetische Zubereitung oder Verwendung nach einem der vorhergehenden Ansprüche, **dadurch gekennzeichnet, dass** das Pfropf-Copolymer ein Acrylat-Rückgrat und Silikon-Seitenketten aufweist.

7. Kosmetische Zubereitung oder Verwendung nach einem der vorhergehenden Ansprüche, **dadurch gekennzeichnet, dass** als Pfropf-Copolymer das Polymer i mit der INCI Acrylates/Dimethicone Copolymer eingesetzt wird.

8. Kosmetische Zubereitung oder Verwendung nach einem der vorhergehenden Ansprüche, **dadurch gekennzeichnet, dass** eines der Silikon-Acrylat-Copolymere ein Copolymer aus (Meth)Acrylsäure und/oder Alkyl(meth)acrylaten mit einem Acrylatester eines dendrimeren Silikons darstellt.

9. Kosmetische Zubereitung oder Verwendung nach einem der vorhergehenden Ansprüche, **dadurch gekennzeichnet, dass** als Copolymer aus (Meth)Acrylsäure und/oder Alkyl(meth)acrylaten mit einem Acrylatester eines dendrimeren Silikon die Verbindung mit der CAS-Nummer 262299-63-8 eingesetzt wird, welche die INCI Acrylates/Polytrimethylsiloxymethacrylate Copolymer trägt.

10. Kosmetische Zubereitung oder Verwendung nach einem der vorhergehenden Ansprüche, **dadurch gekennzeichnet, dass** sie die Silikon/Acrylat-Copolymere mit der INCI Acrylates/Dimethicone Copolymer und Acrytates/Polytrimethylsiloxymethacrylate Copolymer enthält.

11. Kosmetische Zubereitung oder Verwendung nach einem der vorhergehenden Ansprüche, **dadurch gekennzeichnet, dass** sie Cyclomethicone und/oder Isododecan enthält.

12. Kosmetische Zubereitung oder Verwendung nach einem der vorhergehenden Ansprüche, **dadurch gekennzeichnet, dass** sie ein oder mehrere Farbpigmente gewählt aus der Gruppe der Verbindungen mit dem Color Index CI 77491, CI 77492, CI 77499, CI, 77007, CI 77891 enthält.

13. Kosmetische Zubereitung oder Verwendung nach einem der vorhergehenden Ansprüche, **dadurch gekennzeichnet, dass** das Gewichtsverhältnis von Copolymer aus (Meth)Acrylsäure und/oder Alkyl(meth)acrylaten mit einem Acrylatester eines dendrimeren Silikon zu Pfropf-Copolymer 1:4 beträgt.

14. Kosmetische Zubereitung oder Verwendung nach einem der vorhergehenden Ansprüche, **dadurch gekennzeichnet, dass** die Zubereitung W/S-Emulgatoren gewählt aus der Gruppe der Verbindungen Cetyl PEG/PPG-10/1 Dimethicone oder PEG/PPG-19/19 Dimethicone enthält.

15. Kosmetische Zubereitung oder Verwendung nach einem der vorhergehenden Ansprüche, **dadurch gekennzeichnet, dass** die Zubereitung einen oder mehrere Füllstoffe gewählt aus der Gruppe der Verbindungen Disteardimoniumhectorid. Nylon 6/12, Silica, Aluminiumstärkeoctenylsuccinate. Dimethicon-Crosspolymer, Dimethicon/Vinyldimethicon-Crosspolymer, Methylmethacrylat-Crosspolymer, Lauroyl Lysine, Polymethylsilsequioxan enthält.

16. Kosmetische Zubereitung oder Verwendung nach einem der vorhergehenden Ansprüche, **dadurch gekennzeichnet, dass** die Gesamtmenge an Silikon-Acrylat-Copolymeren in der Zubereitung von 0,5 bis 15 Gewichts-%, bezogen auf das Gesamtgewicht der Zubereitung, beträgt.

17. Kosmetische Zubereitung oder Verwendung nach einem der vorhergehenden Ansprüche, **dadurch gekennzeichnet, dass** die Zubereitung eine Foundation darstellt.
